Europäisches Patentamt

European Patent Office

Office européen des brevets

(19)

(11) Veröffentlichungsnummer: **0 051 765**
**B1**

(12) # EUROPÄISCHE PATENTSCHRIFT

(45) Veröffentlichungstag der Patentschrift:
11.09.85

(51) Int. Cl.⁴: **G 01 N 5/04,** G 01 N 33/38

(21) Anmeldenummer: 81108610.7

(22) Anmeldetag: 21.10.81

(54) **Verfahren zur Bestimmung des Wassergehaltes in Frischbeton.**

(30) Priorität: 06.11.80 DE 3041820

(43) Veröffentlichungstag der Anmeldung:
19.05.82 Patentblatt 82/20

(45) Bekanntmachung des Hinweises auf die Patenterteilung:
11.09.85 Patentblatt 85/37

(84) Benannte Vertragsstaaten:
AT BE CH FR GB LI NL

(56) Entgegenhaltungen:
FR - A - 2 149 106
GB - A - 718 444
GB - A - 830 525
US - A - 4 142 403

K.H. HARTGE: "Die physikalische Untersuchung von Böden", 1971, F.Enke Verlag, Seiten 21-30, Stuttgart, DE

(73) Patentinhaber: Lambertus, Eckhard, Akazienweg 6, D-7440 Nürtingen (DE)

(72) Erfinder: Fischer, Willi, Kleine Rathausgasse 3, D-6453 Seligenstadt (DE)
Erfinder: Lambertus, Eckhard, Akazienweg 6, D-7440 Nürtingen (DE)

(74) Vertreter: Leineweber, Jürgen, Dipl.-Phys., Am Heidstamm 78 a, D-5000 Köln 40 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf ein Verfahren zur Bestimmung des Wassergehaltes eines Materials, bei dem eine Materialprobe gewogen, danach unter Wärmezufuhr und Vakuum getrocknet, wieder gewogen und dann aus dem Gewichtsverlust der Wassergehalt bestimmt wird.

Die Festigkeit von Beton ist abhängig vom Mischungsverhältnis und kann nach 28 Tagen Abbindezeit durch einen Druckversuch an Probekörpern ermittelt werden. Die Festigkeit wird durch das Verhältnis Wasser/Zement (Wasserzementwert) bestimmt. Zur Erzielung einer bestimmten Festigkeit muß daher dieses Verhältnis genau eingehalten werden, bzw. bei Beton mit besonderen Eigenschaften darf ein bestimmter Wasserzementwert nicht überschritten werden.

Der Druckversuch ist nur zum Nachweis der Betongüte, nicht aber zur Überwachung des Betoniervorganges einsetzbar. Zur Frischbetonüberwachung beim Erzeuger oder auf der Baustelle ist die Ermittlung des sogenannten WZ-Wertes (Wasserzementwertes) geeignet. Bei Beton B II ist die Prüfung des Wasserzementwertes in einem bestimmten Umfang Vorschrift. Die wichtigste und bisher am meisten angewendete Methode zur Bestimmung dieses Wertes ist das Darr-Verfahren. Bei diesem vorbekannten Verfahren wird eine gewogene, in einem offenen Behälter befindliche Frischbetonprobe z. B. auf einem Gasbrenner möglichst schnell erhitzt und rasch getrocknet. Danach wird der Beton nochmals gewogen und aufgrund des Gewichtsverlustes der WZ-Wert berechnet.

Nachteilig an diesem vorbekannten Verfahren ist, daß durch das Rühren, durch Dampfblasenbildung und auch durch Siedeverzüge, Stoffverluste eintreten, welche die Feststellung des Gewichtsverlustes erheblich beeinträchtigen können. Weiterhin besteht die Gefahr einer ungenügenden Trocknung, da die Feststellung des Trocknungsendzeitpunktes mit Hilfe einer über die Probe gehaltenen Glasplatte ungenau ist. Eine weitere Fehlerquelle ist darin zu sehen, daß durch ein bereits einsetzendes Abbinden der Betonprobe während des Erhitzens ein Teil des Wassers chemisch gebunden wird und nicht mehr verflüchtigen kann. Schließlich erfordert das vorbekannte Verfahren ständig die Anwesenheit einer Person, die die Betonprobe umrühren muß.

Ein Verfahren der eingangs erwähnten Art ist aus der GB-A-718 444 bekannt. Als Material werden Kohle-Proben von jeweils etwa 1 g verwendet. Diese werden jeweils in einen Behälter eingebracht und mit einem Deckel abgedeckt. Danach werden die einzelnen Behälter in Öffnungen eines Metallblocks mit einem Rand eingesetzt. Auf den Rand ist eine Glasplatte auflegbar, so daß ein evakuierbarer Raum entsteht. Dieser Raum wird etwa für 1/2 bis 3/4 h evakuiert bei gleichzeitiger Aufheizung des Metallblocks. Danach wird das gesamte System auf Raumtemperatur gekühlt und belüftet. Die Behälter werden entnommen und der zweiten Wägung zugeführt. Durch die GB-A-830 525 ist schließlich ein Gerät zur Feuchtigkeitsbestimmung bekannt, bei dem kleine Probenmengen mitsamt einer Waage in einen Vakuum-Rezipienten eingebracht werden. Während des Evakuierens wird die Probe mit einer infraroten Lampe getrocknet.

Nachteilig an den durch die GB-Schriften vorbekannten Verfahren ist, daß sie aufgrund der verwendeten extrem kleinen Materialproben nicht für die Bestimmung des WZ-Wertes von Frischbeton geeignet sind. Frischbeton enthält neben den Wasser- und Zementanteilen noch Sand, Kies sowie häufig weitere Zuschläge, so daß eine Probe von 1 g oder gar weniger nicht die charakteristische Zusammensetzung des Frischbetons wiedergeben kann.

Der vorliegenden Erfindung liegt die Aufgabe zugrunde, ein Verfahren anzugeben, mit dem die Bestimmung des Wassergehaltes bzw. des WZ-Wertes von Frischbeton wesentlich einfacher und exakter erfolgen kann.

Erfindungsgemäß wird diese Aufgabe dadurch gelöst, daß das Verfahren der eingangs genannten Art auf Frischbetonproben in einer Menge von mindestens 4000 g angewendet wird. Gegenüber dem Darr-Verfahren ist vorteilhaft, daß in der ersten Phase des Vakuumtrocknungsvorganges trotz hoher Trocknungsgeschwindigkeit (infolge des Absaugens des Wasserdampfes durch die angeschlossene Vakuumpumpe) die Temperatur der Frischbetonprobe unter der Abbindetemperatur gehalten werden kann, da die zur Verdunstung des Wassers notwendige Energie zunächst der Betonprobe in Form von Wärme entzogen wird. Um zu verhindern, daß die Temperatur zu stark absinkt, ist es erforderlich, von außen Wärme zuzuführen. Erst in der zweiten Phase des Vakuumtrocknungsvorganges nimmt die Betonprobe höhere Temperaturen an, wodurch die Restfeuchte quantitativ ausgetrieben wird. In dieser Phase findet praktisch kein Abbinden mehr statt. Da der Behälter, in dem sich die Frischbetonprobe während des Vakuumtrocknungsvorganges befindet, notwendigerweise geschlossen sein muß, können Stoffverluste nicht mehr auftreten und die Messungen verfälschen. Ein ständiges Umrühren der Probe entfällt. Ein weiterer wesentlicher Vorteil besteht darin, daß die zurückbleibende Trockenmasse gegenüber der ursprünglichen Trockenmischung unverändert ist, so daß sich durch nachträgliche Untersuchungen (z. B. durch Siebanalysen) die genauen Zusammensetzungen und die Eigenschaften der Einsatzstoffe (z. B. die Menge und die Eigenschaften des eingesetzten Zements, der Mehlkorngehalt des Frischbetons, die Sieblinie der Zuschläge) ermitteln lassen. Infolge der größeren Prüfgenauigkeit kann dieses Verfahren zur Steuerung und Korrektur des Mischungsverhältnisses bei Lieferbeton und auf der Baustelle dienen. Dadurch kann die Vorhaltung reduziert und neben der Materialeinsparung eine größere Produktionssicherheit erzielt werden.

Die Genauigkeit kann noch erhöht werden, wenn die Frischbetonprobe vorab eingefroren wird, also ein Gefriertrocknungsverfahren angewendet wird. Allerdings erhöht sich dann ebenfalls der Aufwand für die Durchführung des Verfahrens.

Eine Vorrichtung zur Durchführung des beschriebenen Verfahrens umfaßt zweckmäßigerweise einen beheizbaren Behälter, der vakuumdicht verschließbar und an eine Vakuumerzeugungseinrichtung anschließbar ist.

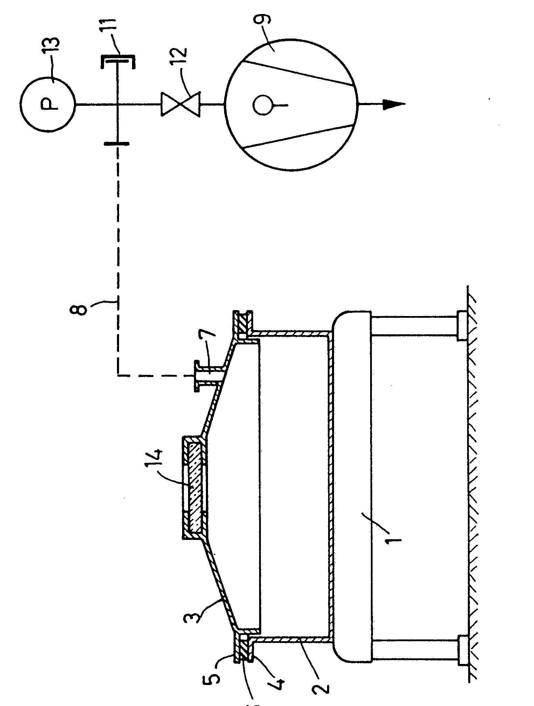

Weitere Vorteile und Einzelheiten der Erfindung sollen anhand eines in der Figur schematisch dargestellten Ausführungsbeispieles erläutert werden.

In der Figur ist mit 1 eine Heizplatte bezeichnet, auf der sich der Behälter 2 für die Frischbetonprobe befindet. Der Behälter 2 ist mit einem Deckel 3 vakuumdicht verschließbar. Dazu weisen Behälter 2 und Deckel 3 einander angepaßte Flansche 4 und 5 auf, zwischen denen sich in geschlossenem Zustand eine Dichtung 6 befindet. Der Deckel 3 ist mit einem Anschlußstutzen 7 ausgerüstet, an den über die Leitung 8 — vorzugsweise eine flexible Leitung, z. B. ein Kunststoff-Schlauch — die Vakuumpumpe 9 angeschlossen ist. In die Leitung 8 sind noch eine Belüftungsklappe 11 und ein Ventil 12 eingeschaltet. Außerdem ist ein Vakuummeter 13 an die Leitung 8 angeschlossen. Weiterhin ist der Deckel 3 noch mit einem Schauglas 14 versehen, so daß die Probe während des Vakuumtrocknungsvorganges beobachtet werden kann.

Zur Durchführung des erfindungsgemäßen Verfahrens wird die Frischbetonprobe, deren Wassergehalt bestimmt werden soll, in den Behälter 2 eingebracht. Die Größe des Behälters soll so bemessen sein, daß mindestens 4 kg Frischbeton darin eingefüllt werden können, da sonst keine exakte Probenzusammensetzung gewährleistet ist. Danach wird der Behälter 2 mit Hilfe des Deckels 3 verschlossen und die bereits warm gefahrene Vakuumpumpe 9 über den Stutzen 7 und die Leitung 8 durch Öffnen des Ventils 12 an den Innenraum des Behälters 2 angeschlossen. Dadurch wird der Vakuumtrocknungsvorgang eingeleitet. Während dieses Trocknungsvorganges wird dem Behälter 2 mit Hilfe der Heizplatte 1 Wärme zugeführt, so daß ein schnelles Verdunsten des in der Probe enthaltenen Wassers gewährleistet ist. Die Leistung der Heizplatte 1 und das Saugvermögen der Vakuumpumpe 9 sind der Größe des Behälters 2 bzw. der üblicherweise in diesem Behälter 2 zu trocknenden Frischbetonmenge anzupassen.

Die Feststellung des Endes des Trocknungsvorganges kann durch Beobachtung des Druckes im Behälter 2 bei geschlossenem Ventil 12, also vom Behälter 2 abgetrennter Vakuumpumpe 9, festgestellt werden. Ist die Betonprobe noch nicht trocken, wird nach dem Schließen des Ventils 12 der Druck im Behälter 2 relativ schnell ansteigen. Tritt praktisch kein Druckanstieg mehr ein, ist der Trocknungsvorgang beendet.

In diesem Fall wird das Ventil 12 geschlossen und der Behälter 2 mit Hilfe der Klappe 11 belüftet, so daß der Deckel 3 abgenommen werden kann.

Zur Feststellung des Gewichtsverlustes sind folgende Werte durch Wägen zu bestimmen:

$G_1$: Leergewicht des Behälters 2,

$G_2$: Gesamtgewicht der Frischbetonprobe und des Behälters 2,

$G_3$: Gesamtgewicht der Trockenmasse und des Behälters 2.

Aus diesen Werten lassen sich dann wie beim vorbekannten Darr-Verfahren der Wassergehalt und der WZ-Wert des Frischbetons berechnen.

Es kann zweckmäßig sein, mit der Zufuhr von Wärme zur Frischbetonprobe erst dann zu beginnen, wenn der Druck im Behälter 2 bereits einen bestimmten Wert (z. B. 100 mbar) erreicht hat. Die vor diesem Zeitpunkt bereits verdunstende Wassermenge kühlt dann infolge der notwendigen Verdunstungsenergie die Frischbetonprobe ab, so daß mit Sicherheit erreicht wird, daß die Temperatur der Frischbetonprobe unter der Abbindetemperatur bleibt.

## Patentansprüche

1. Verfahren zur Bestimmung des Wassergehaltes eines Materials, bei dem eine Materialprobe gewogen, danach unter Wärmezufuhr und Vakuum getrocknet, wieder gewogen und dann aus dem Gewichtsverlust der Wassergehalt bestimmt wird, dadurch gekennzeichnet, daß das Verfahren auf Frischbetonproben in einer Menge von mindestens 4000 g angewendet wird.

2. Verfahren nach Anspruch 1, dadurch gekennzeichnet, daß die Vakuumtrocknung der Frischbetonprobe bei einem Druck kleiner 200 mbar — vorzugsweise kleiner 100 mbar — erfolgt.

3. Verfahren nach Anspruch 1 oder 2, dadurch gekennzeichnet, daß mit der Wärmezufuhr zur Frischbetonprobe erst dann begonnen wird, wenn nach dem Beginn der Vakuumtrocknung ein bestimmter Druckwert erreicht worden ist.

4. Verfahren nach Anspruch 1, 2 oder 3, dadurch gekennzeichnet, daß das Ende der Trocknung durch Beobachtung des Druckes nach einer Abtrennung der Vakuumpumpe festgestellt wird.

5. Verfahren nach Anspruch 1, 2, 3 oder 4, dadurch gekennzeichnet, daß die Frischbetonprobe vor der Vakuumtrocknung eingefroren wird.

## Claims

1. Process for determining the water content of a material, whereby a material specimen is weighed, then heated and vacuum-dried and weighed again, and the water content determined from the weight difference, characterized by the fact that the process can be applied to fresh-concrete specimens with a mass of at least 4000 grams.

2. Process according to claim 1, characterized by the fact that vacuum-drying of the fresh-concrete specimen occurs at pressures smaller than 200 mbar — preferably smaller than 100 mbar.

3. Process according to claims 1 or 2, characterized by the fact that the fresh-concrete specimen is heated only after vacuum-drying has resulted in a specified pressure level.

4. Process according to claims 1, 2 or 3, characterized by the fact that the end of the drying process is determined by watching the pressure build-up after disconnecting the vacuum pump.

5. Process according to claims 1, 2, 3 or 4, characterized by the fact that the fresh-concrete specimen is frozen before being subjected to vacuum-drying.

**Revendications**

1. Procédé pour déterminer la teneur en eau d'un matériau, selon lequel on pèse un échantillon du matériau, on le sèche avec apport de chaleur et sous vide, on le pèse de nouveau puis on détermine la teneur en eau d'après la perte de poids, caractérisé en ce que l'on applique le procédé à des échantillons de béton frais d'au moins 4000 g.

2. Procédé selon la revendication 1, caractérisé en ce que le séchage sous vide de l'échantillon de béton frais s'effectue sous une pression inférieure à 200 mbar, de préférence inférieure à 100 mbar.

3. Procédé selon la revendication 1 ou 2, caractérisé en ce que l'on commence seulement avec l'apport de chaleur à l'échantillon de béton frais lorsque, après le début du séchage sous vide, une valeur de pression déterminée a été atteinte.

4. Procédé selon la revendication 1, 2 ou 3, caractérisé en ce que l'on constate la fin du séchage en observant la pression après une déconnexion de la pompe à vide.

5. Procédé selon la revendication 1, 2, 3 ou 4, caractérisé en ce que l'on produit la congélation de l'échantillon de béton frais avant son séchage sous vide.